# EUROPÄISCHE PATENTANMELDUNG

(11) **EP 0 888 781 A2**
(43) Veröffentlichungstag der Anmeldung: **07.01.1999**
(21) Anmeldenummer: 98112090.0
(22) Anmeldetag: 01.07.1998
(51) Int. Cl.: A61L 9/12

(54) **Halter für einen Duftstoffträger**

(30) Priorität: 02.07.1997 CH 1597/97
(71) Anmelder: Weber Druck + Kartonage AG, 5737 Menziken (CH)
(72) Erfinder: Weber, Markus, 5737 Menziken (CH)
(74) Vertreter: Troesch Scheidegger Werner AG

(57) **Zusammenfassung**

Der Duftstoffträgerhalter weist eine rechteckige Hülle (1), vorzugsweise aus Karton auf, welche an einer Kante (3) eine Einschuböffnung für den scheibenförmigen Duftstoffträger (2) aufweist. Die vorderseitige Hüllenwand (4) weist eine Aussparung (5) auf, welche nur einen Teilabschnitt eines eingeschobenen Duftstoffträgers (2) freigibt, wobei durch Drehen des Duftstoffträgers (2) bei Bedarf ein unverbrauchter Abschnitt vor die Aussparung (5) gebracht werden kann.

## Beschreibung

Die vorliegende Erfindung betrifft einen Halter für einen Duftstoffträger in Form einer kreisförmigen Scheibe.

Die heute bekannten Duftstoffträger werden üblicherweise in Dosen angeboten, welche im Betrieb je nach Bedarf mehr oder weniger geöffnet werden können, oder aber nach Öffnen der Schutzverpackung als Ganzes im Raum aufgestellt werden. Die erste Lösung ist bezüglich Verpackung sehr aufwendig, die zweite erlaubt kein dosiertes Abgeben der Duftstoffe.

Aufgabe der vorliegenden Erfindung war die Schaffung eines Halters für Duftstoffträger, welcher einfach und kostengünstig in der Herstellung ist und trotzdem eine "gesteuerte" Abgabe des Duftstoffes erlaubt.

Erfindungsgemäss wird diese Aufgabe mit einem Halter gelöst, welcher die Merkmale gemäss dem kennzeichnenden Teil von Anspruch 1 aufweist.

Besondere Ausführungsformen des erfindungsgemässen Halters für Duftstoffträger sind in den abhängigen Ansprüchen definiert.

Die Erfindung wird nachstehend anhand von in der Zeichnung dargestellten Ausführungsformen noch etwas näher erläutert. Es zeigen:
- Fig. 1:: eine schaubildliche Darstellung eines Halters nach der Erfindung;
- Fig. 1a:: eine Nabe zur Zentrierung der Trägerscheibe;
- Fig. 2:: eine ähnliche Darstellung wie Fig. 1 eines Halters mit verlängerter Rückwand;
- Fig. 3:: den Halter nach Fig. 2, wie er zum Verkauf angeboten wird und
- Fig. 4:: eine Ansicht des Halters nach Fig. 2 und 3, bereit zum Gebrauch.

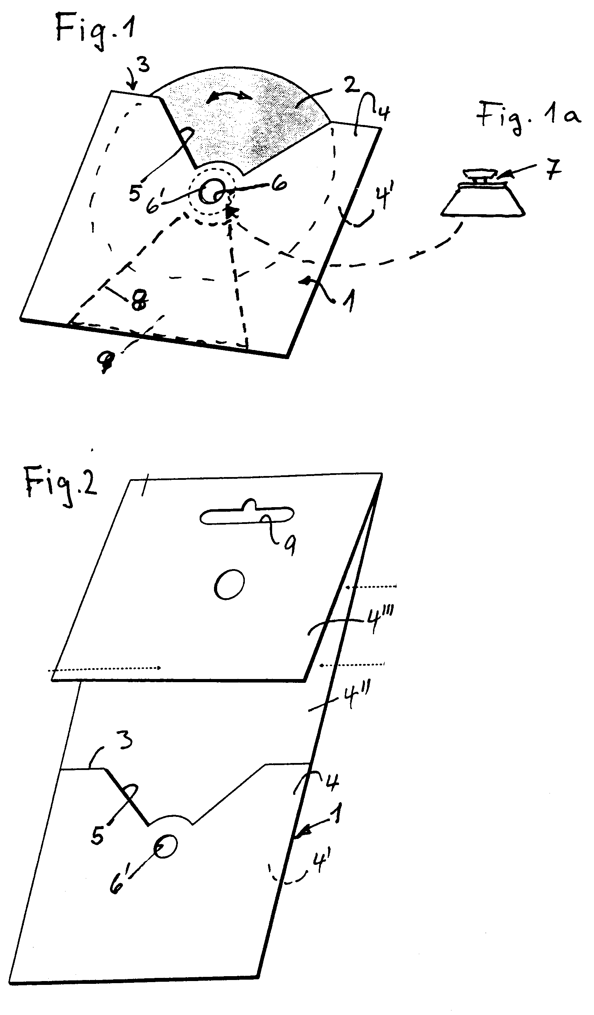

Figur 1 der Zeichnung zeigt einen Halter 1 für einen Duftstoffträger 2 in Form einer kreisförmigen Scheibe. Der Träger besteht aus einem saugfähigen Material (z.B. Zellstoff). Der Halter 1 besteht aus einer Hülle, vorzugsweise aus Karton, und weist an einer Seitenkante 3 eine Einschuböffnung (Schlitz) auf, durch welchen die Scheibe 2 einschiebbar ist.

Die obere bzw. vorderseitige Hüllenwand 4 weist im mittleren Abschnitt der Kante 3 einen sich gegen das Hüllenzentrum hin verengenden Ausschnitt 5 auf, welcher bei eingeschobener Scheibe 2 einen Teilabschnitt (ca. 1/4 der Scheibenfläche) zur Duftabgabe freigibt.

Die Scheibe 2 weist einen Durchmesser auf, welcher etwas kleiner ist als die Hüllenbreite und besitzt im Zentrum ein Loch 6 zu deren drehbaren Zentrierung um eine Nabe 7 (Fig. 1a), welche durch entsprechende Löcher 6' der beiden Seitenwände 4, 4' der Hülle 1 mit dazwischenliegender Scheibe 2 geführt wird und dabei in den Lochrändern einrastet.

Dank dieser Konstruktion des Halters kann jeweils nur ein Teil der Duftstoffträgerscheibe 2 zur Duftstoffabgabe frei bleiben. Bei verbrauchtem Duftstoff kann die Scheibe 2 weitergedreht werden und dabei eine noch genügend Duftstoff aufweisende Teilfläche freigeben.

Die vorderseitige Wand 4 der Hülle 1 kann auf der dem Ausschnitt 5 gegenüberliegenden Seite einen durch Perforationslinien 7 begrenzten Abschnitt 8 aufweisen, welcher abgelöst werden kann, falls die Freigabe einer grösseren Trägerfläche gewünscht würde.

Fig. 2 zeigt eine Variante des Halters nach Fig. 1, bei welcher die Rückwand 4' der Hülle 1 über die Öffnungskante 3 hinaus verlängert und erst noch auf sich zurückgefaltet ist (Wandabschnitte 4'' und 4 ''').

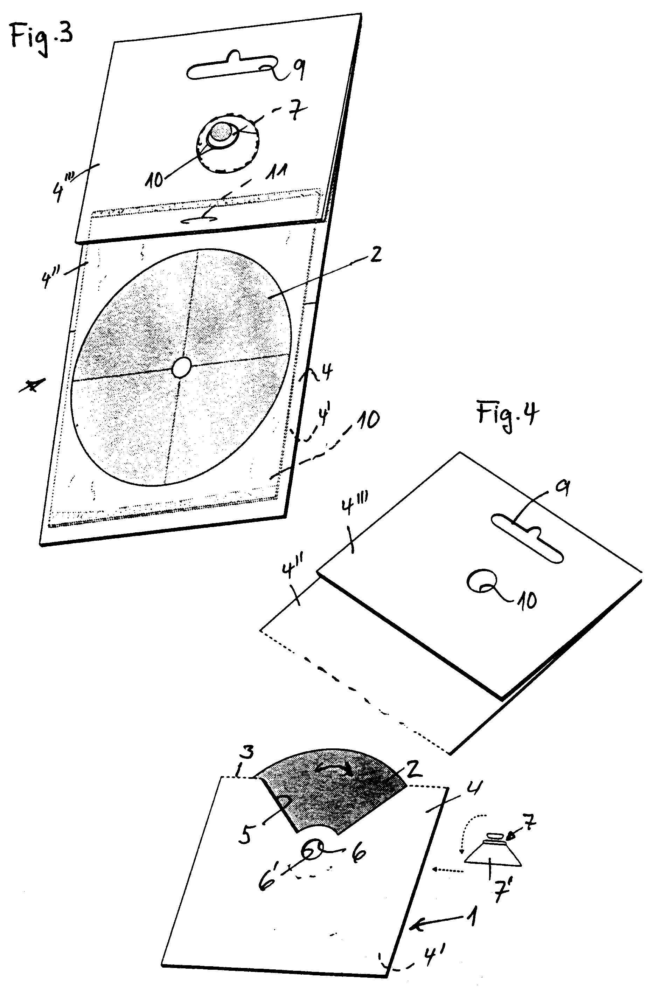

In den Wandteilen 4'' und 4'', sind Öffnungen 9 zum Aufhängen des Halters (insbesondere zur Verkaufspräsentation) bzw. im Wandteil 4''' ein Loch 10 vorgesehen, um die Nabe 7 wie in Fig. 3 gezeigt vor dem Gebrauch unterzubringen. Auch der in eine Schutzhülle 10 verpackte Duftstoffträger 2 wird vor dem Gebrauch z.B. zwischen den Wandteilen 4'' und 4''' z.B. mittels Klammer 11 festgemacht.

Fig. 4 der Zeichnung schliesslich zeigt einen Halter nach Fig. 2 und 3 bereit zum Einsatz: Nach dem Abtrennen (Perforationslinien) der verlängerten Rückwand 4', 4'' ist die Scheibe 2 in die Hülle 1 einzuschieben und die Nabe 7 von hinten durch die Löcher 6, 6' zu drücken.

Die Nabe 7 ist mit Mitteln zu deren Befestigung, z.B. an einer Wand, versehen, z.B. mit einem Sauger 7' (Nabe und Sauger sind dabei vorzugsweise einteilig aus einem elastisch verformbaren Material gefertigt).

## Patentansprüche

1. Halter für einen Duftstoffträger in Form einer kreisförmigen Scheibe, dadurch gekennzeichnet, dass er eine im wesentlichen rechteckige Hülle mit an einer Seitenkante vorgesehener Einschuböffnung für den Duftstoffträger umfasst, wobei die vorderseitige Hüllenwand im mittleren Abschnitt der die Einschuböffnung aufweisenden Kante einen sich zum Wandzentrum hin erstreckenden, sich zunehmend verengenden Ausschnitt aufweist, welcher bei eingeschobenem Duftträger einen Teilabschnitt des letzteren zur Duftabgabe freigibt.

2. Halter nach Anspruch 1, dadurch gekennzeichnet, dass der Ausschnitt so dimensioniert ist, um etwa ¼ einer eingeschobenen Duftstoffträgerscheibe freizugeben.

3. Halter nach einem der Ansprüche 1 oder 2 für eine Duftstoffträgerscheibe mit zentralem Loch, dadurch gekennzeichnet, dass die Hülle im zentralen Wandbereich ein durchgehendes Loch aufweist und dass ferner eine durch das Loch der Hülle und jenes einer eingeschobenen Duftstoffträgerscheibe steckbare, in der Hülle einrastbare Nabe vorgesehen ist, welche zur drehbaren Halterung der Duftstoffscheibe dient und an ihrem rückseitigen Ende mit Befestigungsmitteln versehen ist, um so der Befestigung des Halters zu dienen.

4. Halter nach Anspruch 3, dadurch gekennzeichnet, dass das durch die Hülle fuhrende Loch zwischen Hüllenzentrum und Einschubkante vorgesehen ist, derart, dass eine darin drehbar gehaltene Duftstoffträgerscheibe über die Einschubkante vorsteht.

5. Halter nach Anspruch 3 oder 4, dadurch gekennzeichnet, dass die Befestigungsmittel als Sauger ausgebildet sind, wobei vorzugsweise Nabe und Sauger einstückig aus demselben elastischen Material bestehen.

6. Halter aus Karton nach einem der Ansprüche 1 bis 5, dadurch gekennzeichnet, dass die mit der Aussparung versehene Hüllenwand einen der Aussparung gegenüberliegenden, durch Perforationslinien abgegrenzten Bereich aufweist, welcher zur Erhöhung der freizuhaltenden Fläche einer eingeschobenen Duftstoffträgerscheibe ablösbar ist.

7. Halter nach einem der Ansprüche 1 bis 6, dadurch gekennzeichnet, dass die rückseitige Hüllenwand über die Einschuböffnung hinaus verlängert ist und diese Wandverlängerung gegebenenfalls über eine Perforationslinie von der Hülle abtrennbar ist.

8. Halter nach Anspruch 3 und 7, dadurch gekennzeichnet, dass der von der Hülle entfernt liegende Teil der Wandverlängerung auf sich selbst zurückgefaltet ist und mit einem Loch versehen ist, so dass die Nabe in diesem Loch zwischen den übereinanderliegenden Wandverlängerungsabschnitten vor Gebrauch gehalten werden kann.

9. Halter nach Anspruch 7 oder 8, dadurch gekennzeichnet, dass in der Wandverlängerung eine Öffnung zum Aufhängen des Haltern vorgesehen ist.

10. Halter nach einem der Ansprüche 7 bis 9, dadurch gekennzeichnet, dass eine in eine Schutzhülle verpackte Duftstoffträgerscheibe am oberen Schutzhüllenrand vor Gebrauch zwischen den übereinanderliegenden Wandverlängerungsabschnitten festgehalten ist, z.B. mittels einer Klammer.
